Europäisches Patentamt

European Patent Office

Office européen des brevets

(11) Veröffentlichungsnummer: **0 296 274 B1**

(12) **EUROPÄISCHE PATENTSCHRIFT**

(45) Veröffentlichungstag der Patentschrift: **31.07.91**

(51) Int. Cl.⁵: **A61F 2/34**

(21) Anmeldenummer: **87110063.2**

(22) Anmeldetag: **11.07.87**

(54) **Metallene Aussenschale.**

(30) Priorität: **25.06.87 CH 2398/87**

(43) Veröffentlichungstag der Anmeldung:
**28.12.88 Patentblatt 88/52**

(45) Bekanntmachung des Hinweises auf die
Patenterteilung:
**31.07.91 Patentblatt 91/31**

(84) Benannte Vertragsstaaten:
**AT DE FR GB IT**

(56) Entgegenhaltungen:
**DE-A- 2 314 708**
**FR-A- 2 591 471**
**US-A- 3 685 058**

(73) Patentinhaber: **GEBRÜDER SULZER AKTIENGE-
SELLSCHAFT**
**Zürcherstrasse 9**
**CH-8401 Winterthur(CH)**

(72) Erfinder: **Zweymüller, Karl, Prof. Dr.-med.**
**Orthopädische Universitäts-Klinik Garnisonstr. 13**
**A-1090 Wien(AT)**
Erfinder: **Koch, Rudolph**
**Oberdorfstrasse 229**
**CH-8267 Berlingen(CH)**

## Beschreibung

Die Erfindung betrifft eine metallene Aussenschale für die Aufnahme eines Kunststoff-Pfannenkörpers einer künstlichen Hüftgelenkspfanne zur zementfreie Verankerung im Beckenknochen, wobei die Aussenschale im Scheitelbereich offen ist.

Hüftgelenkspfannen aus einer offenen Aussenschale und einem Kunststoff-Pfannenkörper sind bekannt (FR-A-2 591 471, DE-A 33 42 035); sie sind für eine zementfreie Verankerung im Beckenknochen bestimmt. Die Aussenschale ist dabei als im Scheitel- oder Polbereich offener Ring ausgebildet, um ein Einbringen und Verdichten von autologem oder homologem Knochenmaterial am "Boden" der operativ geschaffenen, die künstliche Hüftgelenkspfanne aufnehmende Ausnehmung im Beckenknochen und ein Hinterfüllen der ringförmigen Aussenschale mit solchem Material zu ermöglichen. Dieses Einbringen von spongiosem Gewebe kann um so leichter erfolgen, je grösser die dafür vorhandenen Oeffnungen sind. Bei der eingangs erwähnten Konstruktion ist die Aussenschale daher, als nur die Mantelflächen des Kunststoff-Pfannenkörpers abdeckender, den ganzen Polbereich freilassender Ring ausgebildet.

In der Praxis hat sich nun gezeigt, das das Implantieren der ringförmigen Ausenschale, die häufig auf ihre Aussenseite mit einem selbstschneidenden Gewinde versehen ist, relativ schwierig ist, da keine sich in der Pfannenachse erstreckende Führung durch ein am Pol oder Scheitel der Aussenschale zentriertes Einsetzinstrument vorhanden ist.

Aufgabe der Erfindung ist es daher, eine Aussenschale zu schaffen, deren Implantation gegenüber bisherigen Konstruktionen erleichtert ist, ohne das das "Einfüllen" von Knochengewebe in dem "Boden"-Bereich der operativen Ausnehmung im Beckenknochen unzulässig erschwert wird.

Erfindungsgemäss wird diese Aufgabe dadurch gelöst, dass der offene Scheitelbereich durch einen einzigen, durch den Pol verlaufenden Steg überbrückt ist, in dessen Zentrum eine Gewindebohrung vorhanden ist.

In die Gewindebohrung im Zentrum der Aussenschale kann ein Einsetzinstrument eingeschraubt werden, durch das die Aussenschale zentriert wird, und dessen Achse mit der Achse der Aussenschale zusammenfällt. Auf diese Weise ist jede Richtungsänderung der Instrumentenachse eine identische Richtungsänderung der Schalenachse, was deren Ausrichtung erheblich erleichtert.

Darüberhinaus kann, insbesondere bei Ausenschalen mit selbstschneidenden Gewinden, die Lage der Schalenachse beim Schneiden des Gewindes laufend kontrolliert und gegebenenfalls korrigiert werden. Die Beschränkung auf einen einzigen Steg schränkt die "offene" Fläche für das Einbringen von Spongiosa nur minimal ein.

Im folgenden wird die Erfindung anhand eines Ausführungsbeispiels im Zusammenhang mit der Zeichnung näher erläutert.

Fig. 1   zeigt, teilweise im Schnitt I-I von Fig. 2, die neue Aussenschale, während

Fig. 2   eine Ansicht von Fig. 1 von unten darstellt.

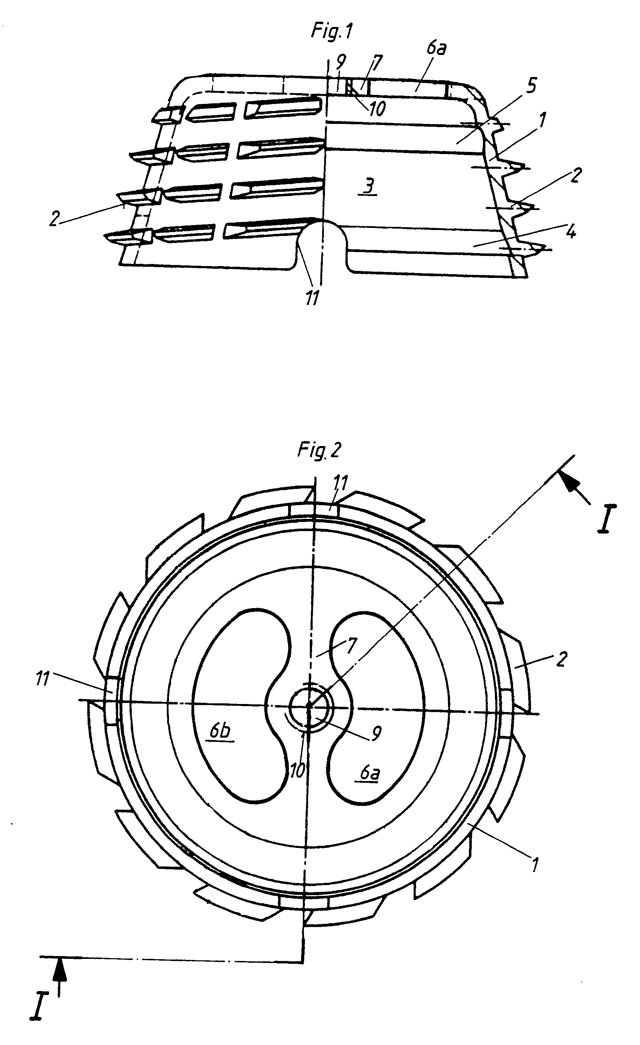

Die in ihrer Grundform kegelstumpfförmige Ausenschale 1, die selbstverständlich auch als Halbkugel ausgebildet sein oder eine andere bekannten Form eine künstlichen Hüftgelenkspfanne haben kann, ist auf ihrer Aussenseite mit einem an sich bekannten selbstschneidenden Gewinde 2 versehen. Sie besteht aus Metall, z.B. aus Titan oder einer Titanlegierung. In ihrem Hohlraum 3 nimmt sie einen nicht gezeigten Pfannenkörper auf, der beispielsweise aus Kunststoff, z.B. aus Polyethylen besteht und die eigentliche Gelenkpfanne enthält, in die ein ebenfalls nicht gezeigter Gelenkkugelkopf einer Femurkopfprothese eingreifen soll.

Die Verbindung der Aussenschale 1 mit dem pfannenkörper erfolgt nahe dem äquatorialen Rand der Prothese mit Hilfe eines bereits früher beschriebenen (EP-A-0245 527) Schnappverschlusses, bei dem aus dem Mantel des Pfannenkörpers in Richtung vom Pol zum Aequator "herauswachsender" Vorsprung in einer als entsprechendes Gegenstück geformte Vertiefung 4 in der Hohlraumwand der Aussenschale einschnappt.

Weiterhin ist polwärts des Schnappverschlusses eine Zylindrische Führung in der Hohlraumwand der Aussenschale 1 vorgesehen. Diese Führung soll bewirken, dass der Pfannenkörper beim Eintreiben oder Einpressen in die bereits im Knochen befestigte Aussenschale 1 geführt wird. Die Führung besteht - wie ebenfalls in der erwähnten EP-Anmeldung beschrieben - aus einem Nokken der in Umfangsrichtung durchgehend aus der Mantelfläche des Pfannenkörpers hervorsteht, und aus einer entsprechenden Nut 5, die in die Wand des Aussenschalhohlraumes 3 eingeschnitten ist.

In dem Beckenknochen zugewandten Pol- oder Scheitelbereich ist die Aussenschale 1 offen. Erfindungsgemäss wird die Oeffnung durch einen Steg 7 zwei, im vorliegenden Beispiel nierenförmige, Teilöffnungen 6a und 6b unterteilt.

Im Zentrum 8 des Steges 7, durch das gleichzeitig die Rotationssymmetrieachse der Aussenschale 1 verläuft, ist eine Bohrung 9 vorgesehen, in die ein Gewinde 10 eingeschnitten ist. Wie bereits erwähnt wird in das Gewinde 10 der Bohrung 9 ein nicht gezeigtes Einsetzinstrument eingeschraubt, mit dem die Aussenschale 1 in den Knochen eingeschraubt werden kann.

Dieses Instrument greift dabei zusätzlich in vier gleichmässig auf den äquatorialen Rand der Aussenschale 1 verteile Ausnehmungen 11 ein. Diese Ausnehmungen 11 dienen zusätzlich als Verdrehsicherung für den Pfannenkörper, der mit zapfenartigen Ansätzen in diese Ausnehmungen 11 eingreift.

**Patentansprüche**

1. Metallene Aussenschale für die Aufnahme eines Kunststoff-Pfannenkörpers einer künstlichen Hüftgelenkspfanne zur zementfreien Verankerung im Beckenknochen, wobei die Aussenschale (1) im Scheitelbereich offen ist, dadurch **gekennzeichnet,** dass der offene Scheitelbereich durch einen einzigen, durch den Pol (8) verlaufenden Steg (7) überbrückt ist, in dessen Zentrum (8) eine Gewindebohrung (9, 10) vorhanden ist.

2. Aussenschale nach Anspruch 1, dadurch gekennzeichnet, dass sie auf ihrer Aussenseite ein selbstschneidendes Gewinde (2) trägt.

**Claims**

1. A metal outer shell adapted to receive a plastics cup of an acetabulum prosthesis for uncemented fixing in the hip bone, the outer shell (1) being open in the apex zone, characterised in that the open apex zone is bridged by a single web (7) which extends through the pole (8) and which is formed at its centre (8) with a tapped bore (9, 10).

2. An outer shell according to claim 1, characterised in that it has a self-tapping screwthread (2) on its outside.

**Revendications**

1. Cupule acétabulaire métallique destinée à loger un corps en matière synthétique d'une cavité cotyloïde artificielle en vue d'un ancrage sans ciment dans l'os iliaque, la cupule acétabulaire (1) étant ouverte dans la région du sommet, caractérisée en ce que la région ouverte du sommet est pontée par une unique entretoise (7) passant par le pôle (8) et au centre (8) de laquelle se trouve un trou taraudé (9, 10).

2. Cupule acétabulaire selon la revendication 1, caractérisée en ce qu'elle comporte du côté extérieur un filetage (2) auto-taraudeur.

Fig.1

Fig. 2